# EUROPÄISCHE PATENTSCHRIFT

(11) **EP 4 563 116 B1**
(45) Veröffentlichungstag und Bekanntmachung des Hinweises auf die Patenterteilung: **09.09.2026**
(21) Anmeldenummer: 23213084.9
(22) Anmeldetag: 29.11.2023
(51) Int. Cl.: A61C 13/00, A61C 13/08, A61C 13/083, A61K 6/818, B33Y 10/00, B33Y 80/00, B33Y 40/20

(54) **SELEKTIVE ANPASSUNG VON HÄRTE UND TRANSLUZENZ BEIM KERAMISCHEN MULTI-COLOR-INKJET-3D-DRUCK**
SELECTIVE ADJUSTMENT OF HARDNESS AND TRANSLUCENCY IN MULTI-COLOR CERAMIC INKJET 3D PRINTING
AJUSTEMENT SÉLECTIF DE DURETÉ ET DE TRANSLUCIDITÉ DANS L'IMPRESSION 3D DE CÉRAMIQUE MULTICOLORE

(43) Veröffentlichungstag der Anmeldung: 04.06.2025
(73) Patentinhaber: Ivoclar Vivadent AG, 9494 Schaan (LI)
(72) Erfinder: John, Hendrik, 9470 Buchs (CH); Rothbrust, Frank, 6822 Röns (AT); Krolikowski, Sebastian, 8805 Richterswil (CH); Ritzberger, Christian, 9472 Grabs (CH)
(74) Vertreter: Baldus, Oliver

(56) Entgegenhaltungen:
- EP-A1- 2 529 694
- DE-A1- 102016 213 243

## Beschreibung

Die vorliegende Erfindung betrifft ein Verfahren zum Herstellen einer dentalen Restauration durch Strahldruck und ein Herstellungsgerät zum Herstellen einer dentalen Restauration durch Strahldruck.

Als Dokumente auf diesem Gebiet werden die EP 2 529 694 A1 und die DE 10 2016 213243 A1 genannt.

Es ist möglich, wässrige oder Lösungsmittel-basierte ZrO2-Schlicker mittels eines Strahldruckens von Material (auch bekannt als Inkjet-Verfahren) schichtweise zu dentalen Restaurationen zu verarbeiten. Durch das Strahldrucken ist es möglich, ortsselektiv unterschiedliche Schlicker aufzutragen. Diese Schlicker können unterschiedlich voreingefärbt sein und zusätzlich unterschiedliche Transluzenzen und/oder unterschiedliche mechanische Eigenschaften haben.

Im Falle von ZrO2-Schlickern können für die verschiedenen Festigkeiten unterschiedliche Yttrium-dotierte Keramikpulver zum Einsatz kommen. Dabei ist die Yttrium-Dotierung auch für den Grad der Transluzenz ursächlich. Die unterschiedlichen Yttrium-dotierten Keramikpulver weisen unterschiedliche Eigenschaften auf. Beispielsweise können drei verschiedene Ausgangspulver oder -Schlicker verwendet werden, um drei Transluzenzstufen abzubilden.

Werden jedoch unterschiedlich voreingefärbte Schlicker verwendet, die alle auch eine unterschiedliche Transluzenz und/oder unterschiedliche mechanische Eigenschaften aufweisen, ist jedem dieser Schlicker ein eigener Druckkopf zugeordnet. Wird beispielsweise ein allgemeines CYMK-Farbschema eingesetzt, um den gesamten Farbraum abzudecken, sind 3 x 4 = 12 Schlicker in 12 Druckköpfen vorzuhalten. Auch die Herstellung dieser verschiedenen Schlicker ist aufwendig. Durch diese große Anzahl an Schlickern ist auch der Bereitstellungs- und Verarbeitungsaufwand entsprechend hoch.

Es ist die technische Aufgabe der vorliegenden Erfindung, eine dentale Restauration mit nur wenigen Basisschlickern, bestenfalls einem einzigen, aufzubauen und die Opazität oder Transluzenz beim Herstellen einer dentalen Restauration örtlich selektiv anzupassen.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Herstellen einer dentalen Restauration durch Strahldruck gelöst, mit den Schritten eines Strahldruckens einer oder mehrerer Schichten der dentalen Restauration mittels eines keramischen Schlickers; und eines Strahldruckens eines Transluzenzverstärkers oder Opazitätsverstärkers auf die eine oder mehreren Schichten. Beim Strahldrucken werden die jeweiligen Materialien durch einen beweglichen Druckkopf tröpfchenweise ortsselektiv ausgestoßen und aufgetragen (Inkjet-Verfahren).

Ein Transluzenzverstärker erhöht die Transluzenz der dentalen Restauration im Sinterverfahren. Demgegenüber erhöht ein Opazitätsverstärker die Opazität der dentalen Restauration im Sinterverfahren. Die reziproke (wechselseitige) Eigenschaft zur Transluzenz ist die Opazität (Lichtundurchlässigkeit).

Durch das Verfahren kann die Anzahl an Druckköpfen eines Herstellungsgerätes verringert werden, ohne in der dentalen Restauration die Freiheitsgrade Farbe, Transluzenz, Opazität und Härte einzuschränken. Dadurch kann der selektive Materialauftrag des Schlickers, die selektive Einfärbung und die selektive Anpassung der Opazität, der Transluzenz und der Härte in voneinander getrennten Prozessschritten durchgeführt werden. Auf diese Weise lassen sich naturgetreue dentale Restaurationen herstellen.

In einer technisch vorteilhaften Ausführungsform des Verfahrens umfasst der Transluzenzverstärker Oxide der Elemente von Yttrium, Lanthan, Ytterbium, Neodym und/oder Europium. Dadurch wird beispielsweise der technische Vorteil erreicht, dass besonders geeignete Transluzenzverstärker verwendet werden.

In einer weiteren technisch vorteilhaften Ausführungsform des Verfahrens umfasst der Opazitätsverstärker Oxide folgender Elemente, wie z.B. Aluminium und/oder Silizium. Dadurch wird beispielsweise der technische Vorteil erreicht, dass besonders geeignete Opazitätsverstärker verwendet werden.

In einer weiteren technisch vorteilhaften Ausführungsform des Verfahrens werden die eine oder mehreren Schichten vor dem Strahldrucken des Transluzenzverstärkers oder Opazitätsverstärkers getrocknet. Dadurch wird beispielsweise der technische Vorteil erreicht, dass ein Verlaufen des Transluzenzverstärkers oder Opazitätsverstärkers vermindert werden kann.

In einer weiteren technisch vorteilhaften Ausführungsform des Verfahrens weist der Schlicker einen Yttrium-Anteil unter 3 mol% auf. Dadurch wird beispielsweise der technische Vorteil erreicht, dass ein opaker Schlicker verwendet wird, der mittels eines Transluzenzverstärkers durchscheinend gemacht werden kann. Auf einen Opazitätsverstärker kann in diesem Fall verzichtet werden.

In einer weiteren technisch vorteilhaften Ausführungsform des Verfahrens weist der Schlicker einen Yttrium-Anteil zwischen 3.5 und 4.5 mol% auf. Dadurch wird beispielsweise der technische Vorteil erreicht, dass sowohl die Transluzenz als auch die Opazität des Schlickers verändert werden können.

In einer weiteren technisch vorteilhaften Ausführungsform des Verfahrens wird eine Färbelösung auf die eine oder mehreren Schichten strahlgedruckt. Dadurch wird beispielsweise der technische Vorteil erreicht, dass die Schichten ortsselektiv eingefärbt werden können.

In einer weiteren technisch vorteilhaften Ausführungsform des Verfahrens ist die Färbelösung mit einem Transluzenzverstärker oder Opazitätsverstärker dotiert. Dadurch wird beispielsweise der technische Vorteil erreicht, dass gleichzeitig beim Färben eine Transluzenz und Opazität eingestellt werden kann.

In einer weiteren technisch vorteilhaften Ausführungsform des Verfahrens wird die hergestellte dentale Restauration vor einem Sinterprozess einem Trocknungs- und/oder Entbinderungsschritt unterzogen. Dieser Trocknungs- und/oder Entbinderungsschritt kann in einem separaten thermischen Prozess erfolgen oder ist ein im Sinterprozess vorgelagerter Prozessschritt. Üblicherweise wird das Trocknen bei Temperaturen von 25°C bis 200°C, bevorzugt 30°C bis 180°C und besonders bevorzugt 40°C bis 150°C durchgeführt. Dabei kann zusätzlich die Luftfeuchtigkeit zwischen 10 bis 90%, bevorzugt 15 bis 85% und besonders bevorzugt 20 bis 80% reguliert werden. Üblicherweise wird bei Temperaturen von 50°C bis 600°C, bevorzugt zwischen 100°C bis 600°C und besonders bevorzugt bei 200°C bis 600°C entbindert. Die Aufheizgeschwindigkeiten liegen zwischen 0.1 bis 10 K/min, bevorzugt zwischen 0.2 bis 10 K/min und besonders bevorzugt zwischen 0.5 bis 10 K/min.

In einer weiteren technisch vorteilhaften Ausführungsform des Verfahrens wird die dentale Restauration in einem Sinterofen gesintert. Dadurch wird beispielsweise der technische Vorteil erreicht, dass eine dentale Restauration mit hoher Festigkeit hergestellt werden kann.

Gemäß einem zweiten Aspekt wird die technische Aufgabe durch ein Herstellungsgerät zum Herstellen einer dentalen Restauration durch Strahldruck gelöst, mit einem ersten Druckkopf zum Strahldrucken einer oder mehrerer Schichten der dentalen Restauration mittels eines keramischen Schlickers; und einem zweiten Druckkopf zum Strahldrucken eines Transluzenzverstärkers oder Opazitätsverstärkers auf die eine oder mehreren Schichten. Durch das Herstellungsgerät werden die gleichen technischen Vorteile wie durch das Verfahren nach dem ersten Aspekt erreicht.

Erfindungsgemäß umfasst das Herstellungsgerät einen Aufnahmebehälter, in dem ein Transluzenzverstärker oder ein Opazitätsverstärker aufgenommen ist. Der jeweilige Aufnahmebehälter kann auswechselbar sein. Dadurch wird beispielsweise der technische Vorteil erreicht, dass der Transluzenzverstärker und/oder Opazitätsverstärker in dem Herstellungsgerät gespeichert ist und auf einfache Weise ausgetauscht werden kann.

In einer weiteren technisch vorteilhaften Ausführungsform des Herstellungsgeräts umfasst das Herstellungsgerät eine Trocknungsvorrichtung zum Trocknen der einen oder mehreren Schichten. Dadurch wird beispielsweise der technische Vorteil erreicht, dass ein Verlaufen des Transluzenzverstärkers und/oder Opazitätsverstärkers vermindert werden kann.

In einer weiteren technisch vorteilhaften Ausführungsform des Herstellungsgeräts umfasst die Trocknungsvorrichtung ein Gebläse und/oder einen Infrarotstrahler. Dadurch wird beispielsweise der technische Vorteil erreicht, dass die Schichten schnell und rissfrei getrocknet werden können.

In einer weiteren technisch vorteilhaften Ausführungsform des Herstellungsgeräts umfasst das Herstellungsgerät ein Dithering-Modul zum Berechnen von Transluzenzzwischenwerten. Dadurch wird beispielsweise der technische Vorteil erreicht, dass die dentale Restauration mit feinabgestimmten Transluzenzwerten erzeugt werden kann.

In einer weiteren technisch vorteilhaften Ausführungsform des Herstellungsgeräts ist das Dithering-Modul ausgebildet, in aufeinanderfolgenden Schichten der dentalen Restauration unterschiedliche zweidimensionale Dithering-Muster zu verwenden. Dadurch wird beispielsweise der technische Vorteil erreicht, dass das Entstehen von Streifen- oder Moiré-Mustern in der dentalen Restauration verhindert wird.

Ausführungsbeispiele der Erfindung sind in den Zeichnungen dargestellt und werden im Folgenden näher beschrieben.

Es zeigen:
- Fig. 1: eine Darstellung unterschiedlicher Bereiche eines Schneidezahns;
- Fig. 2: eine schematische Darstellung einer dentalen Restauration;
- Fig. 3: eine schematische Darstellung eines Herstellungsgeräts zum Herstellen einer dentalen Restauration;
- Fig. 4: eine schematische Darstellung einer Erhöhung der Transluzenz;
- Fig. 5: eine schematische Darstellung einer Erhöhung der Transluzenz oder Erhöhung der Opazität; und
- Fig. 6: ein Blockdiagramm eines Verfahrens zum Herstellen einer dentalen Restauration.

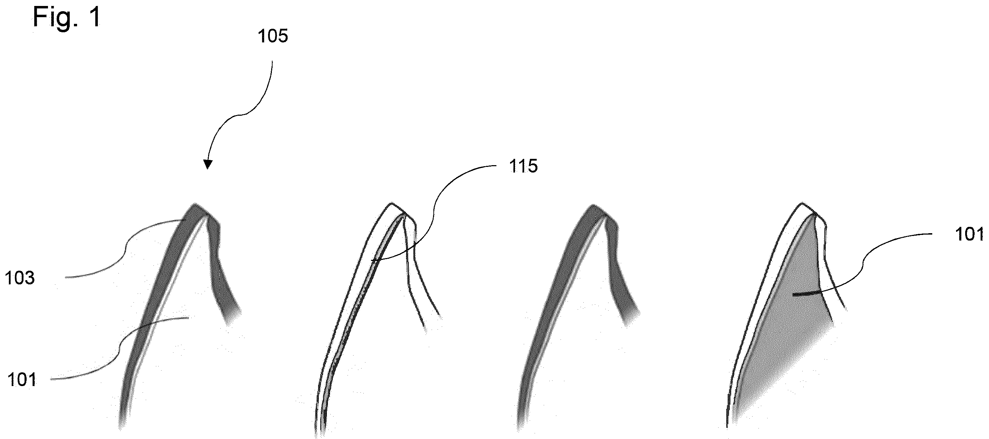

Fig. 1 zeigt eine Darstellung unterschiedlicher Bereiche eines Zahns 105. Der Zahn 105 umfasst einen inneren Dentinkern 101 und einen äußeren Zahnschmelz 103. Für die Grundeinfärbung des Zahns 105 ist der opake, d.h. undurchsichtige Dentinkern 101 verantwortlich. Dieser scheint durch den Zahnschmelz 103 der Schneide hindurch. Der Zahnschmelz 103 ist hingegen oft durchscheinend, d.h. transluzent. Transluzenz ist die partielle Lichtdurchlässigkeit eines Körpers. Um eine dentale Restauration möglichst naturgetreu aussehen zu lassen, wird dieser Aufbau des Zahns 105 auch in künstlichen dentalen Restaurationen verwendet. Hierzu werden Materialien mit unterschiedlichen optischen Eigenschaften bei der Herstellung der dentalen Restauration verwendet.

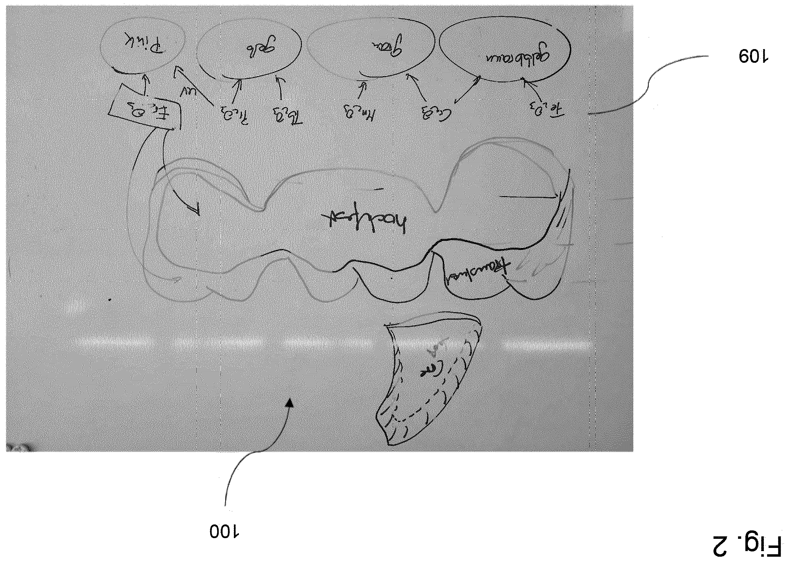

Fig. 2 zeigt eine schematische Darstellung einer dentalen Restauration 100. Die dentale Restauration 100 dient als Zahnersatz und ist beispielsweise durch eine Brücke, Krone, Voll- oder Teilprothese gebildet. Die dentale Restauration 100 wird beispielsweise mittels unterschiedlicher keramischer Schlicker 109 aufgebaut, die Fe2O3, Cr2O3, Mn2O3, Tb2O3, Pr2O3, Er2O3, CeO2, NiO, TiO2 und Co3O4 umfassen. Hierzu werden diese Schlicker 109 bei der räumlichen Herstellung der dentalen Restauration 100 mittels eines Strahldruckverfahrens selektiv in den jeweiligen Bereichen eingesetzt.

Die dentale Restauration 100 wird dabei durch aufeinanderfolgende Schichten aufgebaut, die aufeinander gedruckt werden. Dabei können die Keramikpulver der Schlicker 109 bereits in vorgegebenen Transluzenzen bereitgestellt sein. Durch die Mischung dieser Schlicker 109 ergibt sich die Soll-Transluzenz der dentalen Restauration 100 in dem jeweiligen räumlichen Bereich.

Nach dem selektiven Materialauftrag einer Schicht des Schlickers 109 mittels des Strahldruckverfahrens wird diese Schicht rissfrei getrocknet, indem das Wasser oder das Lösungsmittel als Binder verdampft wird. Zurück bleibt eine poröse Weißling-Schicht, die eine Schichtdicke von 1 um bis 50 um und eine Dichte von mindestens 2.5 g/cm³ aufweist. Dieser Prozess wird wiederholt, bis die gesamte dentale Restauration 100 schichtweise räumlich aufgebaut ist.

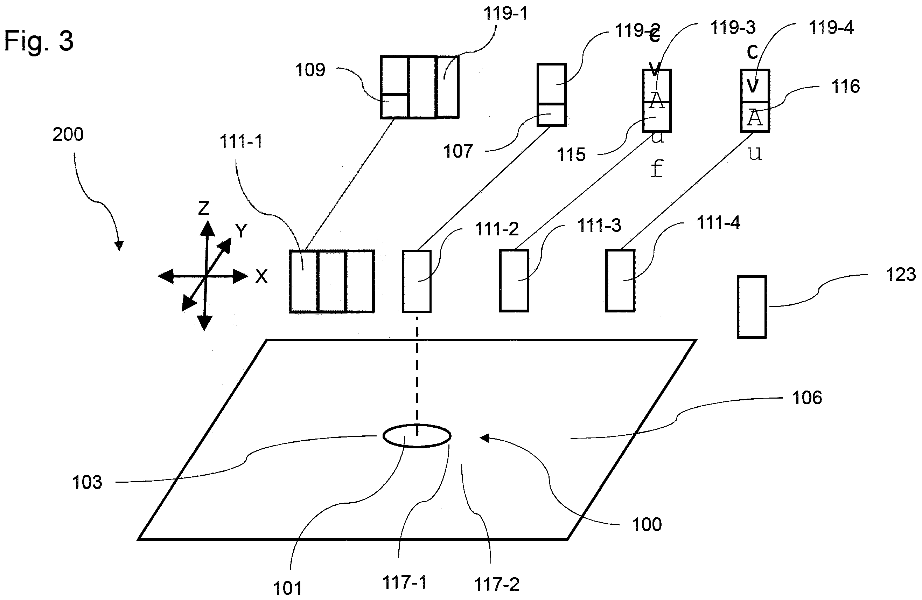

Fig. 3 zeigt eine schematische Darstellung eines Herstellungsgeräts 200 zum Herstellen einer dentalen Restauration 100 durch Strahldruck (Inkjet-Druck) von wässrigen oder Lösungsmittel-basierten Schlickern 109. Um keramische, dentale Multimaterial- und Multicolor-Restaurationen 100 mittels Strahldruck additiv zu fertigen, werden Basis-Schlicker 109 verarbeitet.

Das Herstellungsgerät 200 umfasst eine Mehrzahl von Aufnahmebehältern 119-1, in denen die Basis-Schlicker 109 mit unterschiedlichen optischen Eigenschaften angeordnet sind. Zudem umfasst das Herstellungsgerät 200 zumindest einen Aufnahmebehälter 119-2, in dem ein Transluzenzverstärker oder ein Opazitätsverstärker aufgenommen ist. Zusätzlich umfasst das Herstellungsgerät 200 einen Aufnahmebehälter 119-3 zur Aufnahme einer Färbelösung 115 und einen Aufnahmebehälter 119-4 zur Aufnahme einer Fixierlösung 116.

Die keramischen Schlicker 109 werden jeweils mittels eines zugeordneten Druckkopfes 111-1 tröpfchenweise in mehreren Schichten 117-1, ..., 117-n aufgebracht, um die dentale Restauration 100 schichtweise räumlich aufzubauen. Der Druckkopf und die Bauplattform 106 zum Auftrag des Schlickers sind in 3 Raumachsen (XYZ) relativ zueinander beweglich, so dass der Schlicker 109 an jeder Position aufgedruckt werden kann. Für den selektiven Materialauftrag werden Schlicker 109 mit einem Tropfenvolumen von typischerweise 10 bis 100 pL verwendet. Zum Ausstoßen der Tropfen werden beispielsweise elektrisch gesteuerte Piezoelemente verwendet.

Durch den Einsatz lösungsmittelbasierter oder wässriger Keramikschlicker und die Verdampfung des Lösungsmittels oder Wassers nach Schichtauftrag durch Trocknung entfällt, der zeitlich aufwändige Entbinderungsprozess, der bei Verwendung von Polymeren als Binder erforderlich wäre.

Für die Auftragung des Transluzenzverstärkers oder Opazitätsverstärkers 107 wird ein weiterer Druckkopf 111-2 verwendet, durch den der Transluzenzverstärker oder Opazitätsverstärker 107 auf eine oder mehrere Schichten 117-1, ..., 117-n strahlgedruckt werden kann. Der Druckkopf 111-2 ist ebenfalls in 3 Raumachsen (XYZ) relativ zu der Bauplattform 106 beweglich, so dass die Transluzenzverstärker oder Opazitätsverstärker 107 an jeder Position aufgedruckt werden können.

Für die Auftragung der Färbelösung 115 wird ein weiterer Druckkopf 111-3 verwendet, durch den die Färbelösung 115 auf eine oder mehrere Schichten 117-1, ..., 117-n strahlgedruckt werden kann. Der Druckkopf 111-3 ist ebenfalls in drei Raumachsen (XYZ) relativ zu der Bauplattform 106 beweglich, so dass die Färbelösung an jeder Position aufgedruckt werden können.

Für die Auftragung der Fixierlösung 116, die zur Fixierung der Färbelösung 115 oder des Transluzenzverstärker bzw. Opazitätsverstärkers 107 dient, wird ein weiterer Druckkopf 111-4 verwendet, durch den die Fixierlösung 116 auf eine oder mehrere Schichten 117-1, ..., 117-n strahlgedruckt werden kann. Der Druckkopf 111-4 ist ebenfalls in 3 Raumachsen (XYZ) relativ zu der Bauplattform 106 beweglich, so dass die Färbelösung an jeder Position aufgedruckt werden können.

Die Druckköpfe 111-1, 111-2, 111-3 und 111-4 können unabhängig voneinander steuerbar sein oder in einem gemeinsamen Druckmodul integriert sein.

Selbstverständlich ist ebenfalls möglich, dass die Druckköpfe 111 in Ruhe sind und sich die Bauplattform 106 mit der gedruckten dentalen Restauration 100 und den zuletzt gedruckten Schichten 117 in 3 Richtungen (XYZ) gegenüber den Druckköpfen 111 bewegt, um an jeder Position Schlicker 109, Transluzenzverstärker bzw. Opazitätsverstärkers 107, Färbelösung 115 oder Fixierlösung 116 aufdrucken zu können.

Zum Trocknen des wässrigen Materials ist eine Trocknungsvorrichtung 123 vorgesehen, mit der die Schichten 117-1, ..., 117-n und der Transluzenzverstärker oder Opazitätsverstärker 107 rissfrei getrocknet werden können. Die Trocknungsvorrichtung 123 ist beispielsweise durch ein Gebläse und/oder einen Infrarotstrahler gebildet.

Das Herstellungsgerät 200 stellt eine verringerte Anzahl an voreingefärbten und Yttrium-dotierten neutralen BasisSchlickern 109 bereit, um die Anzahl der Druckköpfe 111-1 und 111-2 zu minimieren und dennoch ein ästhetisches und funktionelles Ergebnis der dentalen Restauration 100 zu erzielen. Dabei wird ein subtraktives Farbsystem verwendet, das einen eingeschränkten, dentalen Farbraum (Dental Color Gamut) aufspannt. Die Farbmischung, das dreidimensionale Halftoning oder Dithering dieser voreingefärbten Schlicker 109 in verschiedenen Verhältnissen entsteht dann in dem speziellen, dentalen Farbraum (Gamut), der die gängigen Zahnfarben, jedoch nicht alle Farben abdeckt.

Bei der Herstellung werden Transluzenzverstärker oder Opazitätsverstärker 107 über einen 3D-Dithering-Algorithmus auf eine Schicht 117-1, ..., 117-n selektiv aufgetragen, der durch ein Dithering-Modul 113 ausgeführt wird. Das Dithering-Modul 113 umfasst hierzu einen Prozessor zum Ausführen des 3D-Dithering-Algorithmus und einen digitalen Datenspeicher zum Speichern der berechneten Mischungsverhältnisse und des Dithering-Algorithmus. Der Prozessor umfasst jedes Hardwaresystem, jede Komponente oder jeden Mechanismus, der Daten, Signale oder andere Informationen verarbeitet. Ein Prozessor kann ein System mit einer zentralen Datenverarbeitungseinheit (CPU), mehreren Datenverarbeitungseinheiten (MPU), eine dedizierte elektrische Schaltung zum Erreichen der Funktionalität oder andere Systeme umfassen. Der Datenspeicher kann Festplatten, Flash-Speicherkarten, wahlfreie Zugriffsspeicher (RAM) oder Nur-Lesespeicher (ROM) umfassen.

Bei dem Dithering werden der Transluzenzverstärker oder Opazitätsverstärker 107 in einem bestimmten Verhältnis und zweidimensionalen Druckmuster in der Druckebene selektiv mittels des Strahldruckverfahrens aufgetragen. Der 3D-Dithering-Algorithmus berechnet zudem, dass nicht gleiche zweidimensionale Dithering-Muster in mehreren Schichten 117-1, ..., 117-n übereinander aufgetragen werden. Dadurch können bei senkrechten Flächen optische Artefakte wie Streifen- oder Moiré-Muster verhindert werden. Die Farbmischung, das dreidimensionale Halftoning oder Dithering dieser voreingefärbten Schlicker 109 in verschiedenen Verhältnissen entsteht innerhalb des speziellen, dentalen Farbraums (Gamut), der die gängigen Zahnfarben, jedoch nicht alle allgemeinen Farben abdeckt.

Bei dem Einsatz von hochviskosen Schlickern 109 mit einer Viskosität von mehr als 100 mPas mit einem hohen Füllgrad werden besondere Anforderungen an den Druckkopf 111-1 und sein Fluidsystem gestellt. Der Druckkopf 111-1 ist mit dem verwendeten Binder und/oder dem Trägermaterial des Schlickers 109 kompatibel, wie beispielsweise Wasser bei wässrigen Schlickern 109, um Korrosion zu vermeiden.

Nach dem selektiven Materialauftrag einer Schicht 117-1, ..., 117-n des Schlickers 109 mittels des Strahldruckverfahrens wird diese Schicht 117-1, ..., 117-n rissfrei getrocknet, indem das Wasser oder das Lösungsmittel als Binder verdampft wird. Dabei kann das Keramikpulver des Schlickers 109 bereits in einer Farbe eingefärbt sein, beispielsweise einer Grundfarbe oder Zahnfarbe. Zurück bleibt eine poröse Weißling-Schicht, die eine Schichtdicke von 1 um bis 50 um und eine Dichte von mindestens 3.0 g/cm³ aufweist. Dieser Prozess wird wiederholt, bis die gesamte dentale Restauration 100 schichtweise räumlich aufgebaut ist.

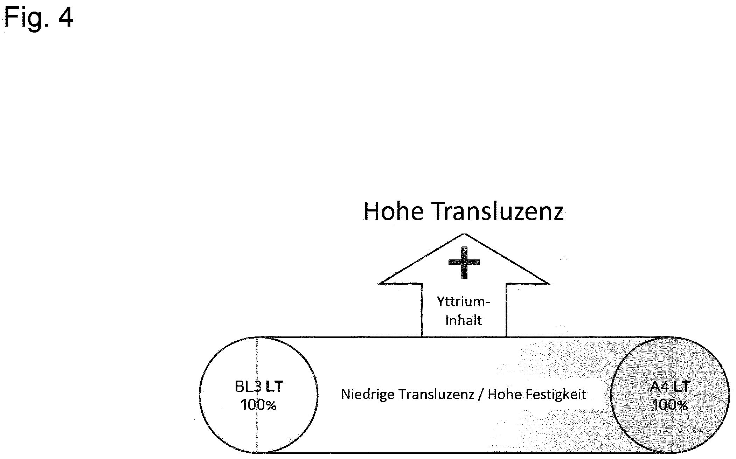

Fig. 4 zeigt eine schematische Darstellung einer Erhöhung der Transluzenz. Die dentale Restauration 100 wird schichtweise aus einem Schlicker 109 aufgebaut, der eine niedrige Transluzenz und hohe Festigkeit aufweist. Die Opazität des Schlickers 109 ist dadurch hoch. Hierzu wird beispielsweise ein Schlicker mit einem Yttrium-Gehalt von 3 mol% Yttrium - 3Y-TZP verwendet. Ausgehend von diesem Schlicker 109 wird die Transluzenz ortsselektiv durch Strahldrucken des Transluzenzverstärkers erhöht. Die Transluzenz kann durch ortsselektives Drucken des Transluzenzverstärkers im Bereich von CR-Wert 90% bis 50% eingestellt werden.

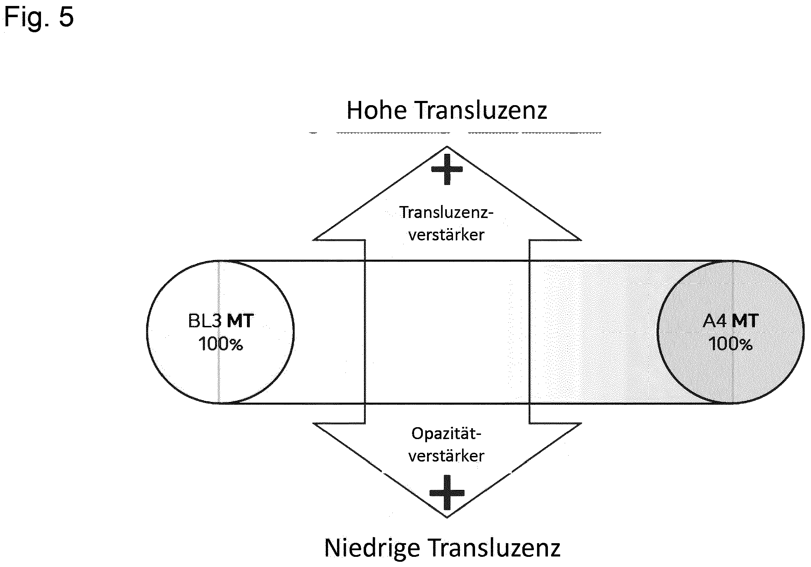

Fig. 5 zeigt eine schematische Darstellung einer Erhöhung der Transluzenz oder Erhöhung der Opazität. Die dentale Restauration 100 wird schichtweise aus einem Schlicker 109 aufgebaut, der eine mittlere Transluzenz oder Opazität aufweist. Hierzu wird beispielsweise ein Schlicker mit einem Yttrium-Gehalt von 4 mol% Yttrium - 4Y-TZP verwendet.

Ausgehend von diesem Schlicker 109 wird die Transluzenz ortsselektiv durch Strahldrucken des Transluzenzverstärkers 107 oder die Opazität durch Strahldrucken eines Opazitätsverstärkers 107 erhöht. Auf diese Weise lassen sich die Transluzenz- oder Opazitätseigenschaften des Schlickers 109 gezielt anpassen.

Neben der örtlichen selektiven Anpassung von Transluzenz, Opazität und Härte während des Schichtaufbaus der dentalen Restauration 100, kann diese auch örtlich selektiv mittels Färbelösungen 115 eingefärbt werden kann. Dies ist dann von Vorteil, wenn nur ein einziger Basisschlicker mit mittlerer Transluzenz in einer Grundfarbe eingesetzt wird. Die Färbelösungen 115 können jeweils bereits mit den geeigneten seltenen Erden dotiert sein, um die erforderliche Anzahl der Druckköpfe 111 noch weiter zu verringern, ohne die Freiheitsgrade der Farbe, Transluzenz, Opazität und Härte einzuschränken.

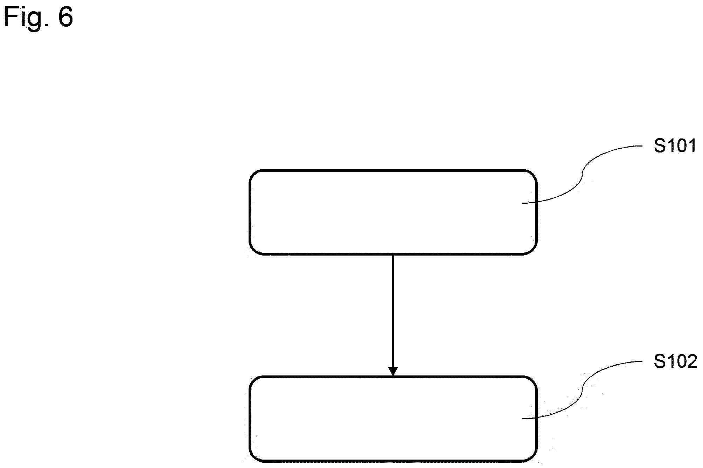

Fig. 6 zeigt ein Blockdiagramm eines Verfahrens zum Herstellen einer dentalen Restauration. In Schritt S101 werden eine oder mehrere Schichten 117-1, ..., 117-n der dentalen Restauration 100 mittels des keramischen Schlickers 109 strahlgedruckt. In Schritt S102 wird ein Transluzenzverstärker oder Opazitätsverstärker 107 auf die eine oder mehreren Schichten 117-1, ..., 117-n strahlgedruckt. Hierbei kann ein einziger Schlicker 109 in einer Grundeinfärbung und einer mittleren Transluzenz, z.B. mit einem Yttrium-Anteil von 4 mol% (4Y-TZP), für den additiven Materialauftrag der dentalen Restauration 100 verwendet werden. Die selektive Einfärbung erfolgt dabei über das Strahldrucken von Färbelösungen 115. Es können Schlicker mit diskreten Partikeln im Nano-(5 nm - 100 nm) oder Submikronbereich (100 nm - 1 µm) oder ionische Lösungen mit hoher Konzentration verwendet werden.

Die selektive Anpassung der Opazität, der Transluzenz und der Härte erfolgt durch den selektiven Auftrag von ionischen Lösungen mit variablen Bestandteilen von Yttrium, Lanthan, Ytterbium und anderen seltenen Erden. Beispielsweise werden Y³⁺, La³⁺, Yb³⁺, Nd³⁺ und Eu³⁺ als Transluzenzverstärker und Al^{3+,} Si als Opazitätsverstärker eingesetzt.

Typischerweise ist der opake Dentinkern für die Farbe verantwortlich und die transluzente Schneide für die Lebendigkeit der dentalen Restauration 100. Mit einem geeignet dotierten Transluzenzverstärker kann beispielsweise der Yttrium-Gehalt von 3Y auf 5Y erhöht werden. Dadurch können ortsselektiv unterschiedliche Opazitätswerte in der dentalen Restauration 100 erreicht werden, wie opake räumliche Bereiche für den Dentinkern und transluzente räumliche Bereiche für die Schneide.

Durch das Mischen von zwei voreingefärbten Schlickern 109 mit heller und dunkler Zahnfarbe in mittlerer Transluzenz und Opazität und dem selektiven Hinzufügen von einem Transluzenzverstärker oder Opazitätsverstärker lassen sich hochästhetische und funktionale dentale Restaurationen 100 erzeugen. Beispielsweise können zwei hochfeste und opake Schlicker für den Dentinkern gemischt werden und ein Transluzenzverstärker in entsprechenden Bereichen für eine weniger feste, transluzente Schneide hinzugefügt werden. In diesem Fall werden nur 4 Druckköpfe benötigt:
- 1: Support Material
- 2: 3Y-TZP Schlicker - hell (opak, hochfest)
- 3: 3Y-TZP Schlicker - dunkel (opak, hochfest)
- 4: Yttrium-Lösung (zur lokalen Steuerung der Transluzenz und Festigkeit)

Alternativ könnte die dentale Restauration mit einem mitteltransluzenten Schlicker 109 aufgebaut werden, dem jeweils selektiv ein Transluzenzverstärker (Translucency Enhancer) oder ein Opazitätsverstärker (Opaquer Liquid) zugefügt wird.

Das selektive Hinzufügen des Transluzenzverstärkers oder Opazitätsverstärkers kann auf die nasse strahlgedruckte Schicht 117-1, ..., 117-n (nass auf nass) oder auf die trockene strahlgedruckte Schicht 117-1, ..., 117-n (nass auf trocken) erfolgen. Die chemische Umsetzung erfolgt während des anschließenden Sinterprozesses des selektiv dotierten Grünlings. Dabei entstehen die gewünschten optischen Eigenschaften.

### BEZUGSZEICHENLISTE

- 100: Dentale Restauration
- 101: Dentinkern
- 103: Zahnschmelz
- 105: Zahn
- 106: Bauplattform
- 107: Transluzenzverstärker/Opazitätsverstärker
- 109: Schlicker
- 111: Druckkopf
- 113: Dithering-Modul
- 115: Färbelösung
- 116: Fixierlösung
- 117: Schicht
- 119: Aufnahmebehälter
- 121: Auftragungsvorrichtung
- 123: Trocknungsvorrichtung

## Patentansprüche

1. Verfahren zum Herstellen einer dentalen Restauration (100) durch Strahldruck, mit den Schritten:
- Strahldrucken (S101) einer oder mehrerer Schichten (117-1, ..., 117-n) der dentalen Restauration (100) mittels eines keramischen Schlickers (109);
**gekennzeichnet durch**
- Strahldrucken (S102) eines Transluzenzverstärkers oder Opazitätsverstärkers (107) auf die eine oder mehreren Schichten (117-1, ..., 117-n).

2. Verfahren nach Anspruch 1, wobei der Transluzenzverstärker Oxide der Elemente von Yttrium, Lanthan, Ytterbium, Neodym und/oder Europium umfasst.

3. Verfahren nach einem der vorhergehenden Ansprüche, wobei der Opazitätsverstärker Aluminium und/oder Silizium umfasst.

4. Verfahren nach einem der vorhergehenden Ansprüche, wobei die eine oder mehreren Schichten (117-1, ..., 117-n) vor dem Strahldrucken des Transluzenzverstärkers oder Opazitätsverstärkers (107) getrocknet werden.

5. Verfahren nach einem der vorhergehenden Ansprüche, wobei der Schlicker 109 einen Yttrium-Anteil unter 3 mol% aufweist.

6. Verfahren nach einem der vorhergehenden Ansprüche, wobei der Schlicker 109 einen Yttrium-Anteil zwischen 3.5 und 4.5 mol% aufweist.

7. Verfahren nach einem der vorhergehenden Ansprüche, wobei eine Färbelösung auf die eine oder mehreren Schichten (117-1, ..., 117-n) strahlgedruckt wird.

8. Verfahren nach einem der vorhergehenden Ansprüche, wobei die Färbelösung mit einem Transluzenzverstärker oder Opazitätsverstärker dotiert ist.

9. Verfahren nach einem der vorhergehenden Ansprüche, wobei die hergestellte dentale Restauration (100) vor einem Sinterprozess einem Trocknungs- und/oder Entbinderungsschritt unterzogen wird.

10. Herstellungsgerät (200) zum Herstellen einer dentalen Restauration (100) durch Strahldruck, mit:
- einem ersten Druckkopf (111-1) zum Strahldrucken einer oder mehrerer Schichten (117-1, ..., 117-n) der dentalen Restauration (100) mittels eines keramischen Schlickers (109); und
- einem zweiten Druckkopf (111-2) zum Strahldrucken eines Transluzenzverstärkers oder Opazitätsverstärkers (107) auf die eine oder mehreren Schichten (117-1, ..., 117-n);
**gekennzeichnet durch**
e inen Aufnahmebehälter (119-3), in dem ein Transluzenzverstärker oder ein Opazitätsverstärker aufgenommen ist.

11. Herstellungsgerät (200) nach Anspruch 10, wobei das Herstellungsgerät (200) eine Trocknungsvorrichtung (123) zum Trocknen der einen oder mehreren Schichten (117-1, ..., 117-n) umfasst.

12. Herstellungsgerät (200) nach einem der Ansprüche 10 oder 11, wobei die Trocknungsvorrichtung (123) ein Gebläse und/oder einen Infrarotstrahler umfasst.

13. Herstellungsgerät (200) nach einem der Ansprüche 10 bis 12, wobei das Herstellungsgerät (200) ein Dithering-Modul (113) zum Berechnen von Transluzenzzwischenwerten umfasst.

14. Herstellungsgerät (200) nach einem der Ansprüche 10 bis 13, wobei das Dithering-Modul (113) ausgebildet ist, in aufeinanderfolgenden Schichten der dentalen Restauration (100) unterschiedliche zweidimensionale Dithering-Muster zu verwenden.

## Claims

1. A method of producing a dental restoration (100) by jet-printing, comprising:
- jet-printing (S101) one or more layers (117-1, ..., 117-n) of the dental restoration (100) using a ceramic slurry (109);
**characterized by**
- jet-printing (S102) a translucency enhancer or opacity enhancer (107) onto the one or more layers (117-1, ..., 117-n).

2. The method according to claim 1, wherein the translucency enhancer comprises oxides of the elements of yttrium, lanthanum, ytterbium, neodymium and/or europium.

3. The method according to any one of the preceding claims, wherein the opacity enhancer comprises aluminum and/or silicon.

4. The method according to any one of the preceding claims, wherein the one or more layers (117-1, ..., 117-n) are dried prior to the jet-printing of the translucency enhancer or opacity enhancer (107).

5. The method according to any one of the preceding claims, wherein the slurry (109) has an yttrium content of less than 3 mol%.

6. The method according to any one of the preceding claims, wherein the slurry (109) has an yttrium content of between 3.5 and 4.5 mol%.

7. The method according to any one of the preceding claims, wherein a coloring solution is jet-printed onto the one or more layers (117-1, ..., 117-n).

8. The method according to any one of the preceding claims, wherein the coloring solution is doped with a translucency enhancer or opacity enhancer.

9. The method according to any one of the preceding claims, wherein the produced dental restoration (100) is subjected to a drying and/or debinding step prior to a sintering process.

10. A production apparatus (200) for producing a dental restoration (100) by jet-printing, comprising:
- a first print head (111-1) for jet-printing one or more layers (117-1, ..., 117-n) of the dental restoration (100) using a ceramic slurry (109); and
- a second print head (111-2) for jet-printing a translucency enhancer or opacity enhancer (107) onto the one or more layers (117-1, ..., 117-n);
**characterized by**
- a receiving container (119-3) which receives a translucency enhancer and/or opacity enhancer.

11. The production apparatus (200) according to claim 10, wherein the production apparatus (200) comprises a drying device (123) for drying the one or more layers (117-1, ..., 117-n).

12. The production apparatus (200) according to any one of claims 10 or 11, wherein the drying device (123) comprises a blower and/or an infrared emitter.

13. The production apparatus (200) according to any one of claims 10 to 12, wherein the production apparatus (200) comprises a dithering module (113) for calculating intermediate translucency values.

14. The production apparatus (200) according to any one of claims 10 to 13, wherein the dithering module (113) is configured to use different two-dimensional dithering patterns in successive layers of the dental restoration (100).

## Revendications

1. Procédé de fabrication d'une restauration dentaire (100) par impression par jet, comprenant les étapes suivantes :
- l'impression par jet (S101) d'une ou plusieurs couches (117-1, ..., 117-n) de la restauration dentaire (100) au moyen d'une barbotine céramique (109) ;
**caractérisé par**
- impression par jet (S102) d'un agent de renforcement de la translucidité ou d'un agent de renforcement de l'opacité (107) sur ladite une ou lesdites plusieurs couches (117-1, ..., 117-n).

2. Procédé selon la revendication 1, où l'agent de renforcement de la translucidité comprend des oxydes des éléments yttrium, lanthane, ytterbium, néodyme et/ou europium.

3. Procédé selon l'une des revendications précédentes, où l'agent de renforcement de l'opacité comprend de l'aluminium et/ou du silicium.

4. Procédé selon l'une des revendications précédentes, où ladite une ou lesdites plusieurs couches (117-1, ..., 117-n) sont séchées avant l'impression par jet de l'agent de renforcement de la translucidité ou de l'agent de renforcement de l'opacité (107).

5. Procédé selon l'une des revendications précédentes, où la barbotine (109) présente une teneur en yttrium inférieure à 3 % molaire.

6. Procédé selon l'une des revendications précédentes, où la barbotine (109) présente une teneur en yttrium comprise entre 3,5 et 4,5 % molaire.

7. Procédé selon l'une des revendications précédentes, où une solution colorante est imprimée par jet sur ladite une ou lesdites plusieurs couches (117-1, ..., 117-n).

8. Procédé selon l'une des revendications précédentes, où la solution colorante est dopée avec un agent de renforcement de la translucidité ou un agent de renforcement de l'opacité.

9. Procédé selon l'une des revendications précédentes, où la restauration dentaire (100) fabriquée est soumise, avant un processus de frittage, à une étape de séchage et/ou de déliantage.

10. Dispositif de fabrication (200) pour fabriquer une restauration dentaire (100) par impression par jet, comprenant :
- une première tête d'impression (111-1) destinée à imprimer par jet une ou plusieurs couches (117-1, ..., 117-n) de la restauration dentaire (100) au moyen d'une barbotine céramique (109) ; et
- une deuxième tête d'impression (111-2) destinée à imprimer par jet un agent de renforcement de la translucidité ou un agent de renforcement de l'opacité (107) sur ladite une ou lesdites plusieurs couches (117-1, ..., 117-n) ;
**caractérisé par**
- un réservoir de réception (119-3), où est logé un agent de renforcement de la translucidité ou un agent de renforcement de l'opacité.

11. Dispositif de fabrication (200) selon la revendication 10, où le dispositif de fabrication (200) comprend un dispositif de séchage (123) destiné à sécher ladite une ou lesdites plusieurs couches (117-1, ..., 117-n).

12. Dispositif de fabrication (200) selon l'une des revendications 10 ou 11, où le dispositif de séchage (123) comprend une soufflerie et/ou un radiateur infrarouge.

13. Dispositif de fabrication (200) selon l'une des revendications 10 à 12, où le dispositif de fabrication (200) comprend un module de tramage (113) destiné à calculer des valeurs intermédiaires de translucidité.

14. Dispositif de fabrication (200) selon l'une des revendications 10 à 13, où le module de tramage (113) est configuré pour utiliser différents motifs de tramage bidimensionnels dans des couches successives de la restauration dentaire (100).
